# EUROPÄISCHE PATENTSCHRIFT

(11) **EP 1 385 505 B1**
(45) Veröffentlichungstag und Bekanntmachung des Hinweises auf die Patenterteilung: **05.07.2006**
(21) Anmeldenummer: 02730223.1
(22) Anmeldetag: 29.04.2002
(51) Int. Cl.: A61K 31/381, A61P 17/00

(54) **BEHANDLUNG DER AKNE MIT LIPOXYGENASE INHIBITOREN**
ACNE TREATMENT WITH LIPOOXIGENASE INHIBITORS
TRAITEMENT DE L'ACNE PAR DES INHIBITEURS DE LIPOXYGENASE

(30) Priorität: 30.04.2001 DE 10121252
(43) Veröffentlichungstag der Anmeldung: 04.02.2004
(73) Patentinhaber: Zouboulis, Christos, 12200 Berlin (DE)
(72) Erfinder: Zouboulis, Christos, 12200 Berlin (DE)
(74) Vertreter: TBK-Patent
(86) Internationale Anmeldenummer: PCT/EP2002/004715
(87) Internationale Veröffentlichungsnummer: WO 2002/089791

(56) Entgegenhaltungen:
- EP-A- 1 176 140
- WO-A-00/23071
- WO-A-97/29776
- US-A- 5 208 251
- US-A- 5 250 565
- US-A- 6 120 779
- HUANG, MOU-TUAN ET AL: "Anti-tumor and anti-carcinogenic activities of triterpenoid,.beta.- boswellic acid" BIOFACTORS (2000), 13(1-4), 225-230 , XP001113014
- SEEGERS, BIANCA A. M. P. A. ET AL: "Pharmacological effects of a specific leukotriene B4 receptor antagonist (VML 295) on blood leukocytes, cutaneous inflammation and epidermal proliferation" SKIN PHARMACOLOGY AND APPLIED SKIN PHYSIOLOGY (2000), 13(2), 75-85 , XP008017148
- MANZ B ET AL: "ANCA-positive vasculitis of the skin and kidneys associated with acne conglobata" HAUTARZT 2002 GERMANY, Bd. 53, Nr. 11, 2002, Seiten 730-734, XP008017011 ISSN: 0017-8470
- DATABASE CA [Online] CHEMICAL ABSTRACTS SERVICE, COLUMBUS, OHIO, US; CONESE, M. ET AL: "Inhibitory effect of retinoids on the generation of procoagulant activity by blood mononuclear phagocytes" retrieved from STN Database accession no. 116:207492 XP002241309 & THROMBOSIS AND HAEMOSTASIS (1991), 66(6), 662-5 ,

## Beschreibung

Die vorliegende Erfindung betrifft die Verwendung wirksamer Substanzen zur Behandlung der Akne, insbesondere der entzündlichen Akne. Die Wirkstoffe können in einer pharmazeutischen Zusammensetzung nach einem beliebigen herkömmlichen Applikationsweg verabreicht werden.

Es wird angenommen, dass Akne-Läsionen aufgrund der Interaktion von miteinander in Beziehung stehenden, komplexen Vorgängen entstehen: einer erhöhten Exkretion des Sebums (Cunliffe WJ, Shuster S: Pathogenesis of acne. Lancet. 1969; 1(7597): 685-687), einer duktalen Kornifizierung (Holmes RL, Williams M, Cunliffe WJ: Pilo-sebaceous duct obstruction and acne. Br J Dermatol. 1972; 87: 327-332 und 35), einer abnormalen Zahl und Funktion von P. acnes (Cunliffe WJ, Clayden AD, Gould D, Simpson NB: Acne vulgaris-its aetiology and treatment. A review. Clin Exp Dermatol. 1981; 6: 461-469) sowie die Produktion entzündlicher Mediatoren, die zur Bildung von Papeln, Pusteln und zeitweise tiefen entzündlichen Läsionen führen.

Die Initiatoren bzw. wirklichen Ursachen der Entzündungsantwort bei der Akne sind jedoch bis heute unklar. Mehrere Faktoren sind als Mediatoren der Entzündung in Betracht gezogen worden. Dies gilt insbesondere für Mikroorganismen, vor allem P. *acnes* (Cunliffe WJ: Acne. London, Dunitz, 1989), sowie deren Produkte (Hellgren L, Selstam G, Vincent J: Prostaglandin-like substances in Propionibacterium acnes.II. Stimulatory effect on ovarian cyclic AMP. Experientia. 1979; 35: 196-197 und Webster GF, Leyden JJ: Characterisation of serum independent polymorphonuclear leucocyte chemotactic factors produced by Propionibacteria acnes. Inflammation 1980; 4: 261-269), wie freie Fettsäuren (die durch einen mittels follikulärer Bakterien initiierten Triglycerid-Metabolismus erzeugt werden können) und oxidative Produkte von Squalen. Dementsprechend bauen herkömmliche Therapiekonzepte gegen Akne in erster Linie auf topischen oder systemischen Antibiotikamitteln auf, die zur Reduzierung der Follikelzahl von Bakterien führen (Hubbell CG, Hobbs ER, Rist T, White JW: Efficacy of minocycline compared with tetracycline in treatment of acne vulgaris. Arch Dermatol. 1982; 118: 989-992; Juhlin L, Liden S: A quantitative evaluation of the effect of oxytetracycline and doxycycline in acne vulgaris. Br J Dermatol. 1969; 81: 154-158 und Leyden JJ, McGinley KJ, Kligman AM: Tetracycline and minocycline treatment. Arch Dermatol. 1982; 118: 19-22). Darüber hinaus sind bisher selten entzündungshemmende Mittel zur topischen oder systemischen Behandlung unterschiedlicher Arten von Akne eingesetzt worden, wie Corticosteroide, Colchizin, Resorchinol, Isoniazid, topisches UV-Licht sowie PUVA (Cunliffe WJ: Acne. London, Dunitz, 1989).

Vor längerer Zeit wurde Benoxaprofen als anti-inflammatorisches Mittel zur Behandlung der nodulären Akne mittels oraler Gabe beschrieben (Cunliffe WJ: Acne. London, Dunitz, 1989). In klinischen Versuchen wurden jedoch nachteilige Wirkungen, einschließlich einer Lichtempfindlichkeit, Hautausschlag, Milien, Onycholysis sowie eine Hepatotoxizität mit Gelbsucht, insbesondere bei älteren Patienten mit beeinträchtigter Nierenfunktion beobachtet, so dass das Medikament schließlich vom Markt genommen wurde (Halsey JP, Cardoe N: Benoxaprofen: Side effect profile in 300 patients. Br Med J. 1982; 284: 1365-1368 und Hindson TC, Daymond T, Diffey B, Lawlor F: Side effects of benoxaprofen. Br Med J Clin Res Ed. 1982; 284: 1368-1369).

Die nachfolgend genannten Dokumente beschreiben bestimmte Substanzen bzw. Verbindungen mit einer Reihe von Wirkmechanismen, u.a. einer 5-Lipoxygenasehemmung, wobei neben einer Vielzahl von Behandlungsmöglichkeiten auch die Akne kurz erwähnt ist:
WO-A-200 105 780 (Chroman-Analoga), WO-A-0 071 540 (Hydrochlorid von 5-(4-(6-Methoxy-1-methyl-1H-benzimidazol-2-yl-methoxy)benzyl)thiazolidin-2,4-dion zur Behandlung von Diabetes), WO-A-0 061 582 (kondensierte Imidazolverbindungen), WO-A-0 061 581 (Benzimidazol- oder Imidazolpyridin-Derivate), WO-A-0 059 889 (Verwendung von α-substituierten Deriväten), WO-A-9 937 314 (lipidhaltiger Extrakt aus der Seegurke), WO-A-9 918 081 (an Phenyl(oxy oder thio)alkylsubstituierte Benzo- oder Pyrido-kondensierte Imidazolverbindungen), US-A-5 196 431 (2-substituierte Amino-4,6-di-tert-butyl-5-hydroxy-pyridinderivate), US-A-5 187 175 (Acyl- oder Hydroxyl-Imino-substituierte Hydroxypyrimidin-Derivate), US-A-5 142 095 (Diaryl-alkanoide bestimmter Struktur), WO-A-92 13 844 (2-substituierte 4,6-di-tert-butyl-5-Hydroxy-1,3-pyrimidin-Verbindungen) und EP-A-449 216 (2-substituierte Azolidinon-Derivate). Ein direkter Zusammenhang zwischen einem spezifischen Lipoxygenase-Inhibitor und einer tatsächlichen therapeutischen Wirksamkeit gegen Akne, speziell beim Menschen, wird in den genannten Dokumenten jedoch nicht angesprochen.

Die WO-A-97/29776 beschreibt die Verwendung einer Kombination eines Cyclooxygenase-2-Inhibitors und eines 5-Lipoxygenase-Inhibitors zur Verhinderung der Abstoßung von Transplantaten und bei der Behandlung von Autoimmunkrankheiten.

Die US-A-5,250,565 und die US-A-5,208,251 beschreiben Wirkstoffe mit 5-Lipoxygenase-Inhibitoraktivität. Pauschal wird dort die Möglichkeit der Behandlung von Akne erwähnt.

Die US-A-6,120,779 beschreibt kosmetische Zubereitungen zur Behandlung von trockener Haut, Haar und anderem keratinisierten Gewebe. Hierzu werden teilweise Cholinsalze von Mono-, Di- und Polycarbonsäuren und deren Hydroxyderivaten als wirksam beschrieben. Boswelliasäure wird als wirksame Komponente zur Behandlung von Akne beschrieben.

Die Aufgabe der Erfindung bestand somit darin, die Therapiemöglichkeiten gegen Akne, insbesondere gegen entzündliche Akne, zu erweitern und dabei die Wirkung unter gleichzeitiger Reduzierung von Nebenwirkungen möglichst zu verbessern.

Die Aufgabe wird erfindungsgemäß durch Verwendung mindestens einer der in Anspruch 1 genannten Substanzen, die aus der aus 5-Lipoxygenase-Inhibitoren bestehenden Gruppe ausgewählt wird zur Behandlung von Akne gelöst. In einem weiteren Gegenstand der Erfindung wird mindestens einer 5-Lipoxigenase-Inhibitoren in einer geeigneten pharmazeutischen Zusammensetzung mit üblichen pharmazeutisch akzeptablen Trägern und ggf. weiteren Hilfsstoffen eingesetzt.

Im Rahmen vorliegender Erfindung wurde überraschender Weise gefunden, dass, entgegen der herrschenden Meinung der Rolle von P. *acnes* oder anderer Mikroorganismen als Hauptursachen-Faktor für die Akne, offenbar intrinsische Mechanismen humaner Sebozyten einen signifikanten Einfluss auf die

Bildung der Akne, speziell der entzündlichen Akne, ausüben können. Es wurde weiterhin gefunden, dass Leukotriene vom Typ B₄ (LTB₄), die 5-Lipoxygenase-Metabolite der Araichidonsäure darstellen, starke Einflussfaktoren sind, was vermutlich auf deren Rolle als Ligand der Peroxysomen-Proliferatoraktivierten Rezeptoren (PPARs), die an der Differenzierung der Talgdrüsenzellen beteiligt sind, zurückzuführen ist. Es kann ferner vermutet werden, dass die Reduzierung der Expression und Proteinbildung proinflammatorischer Zytokine, insbesondere des Zytokins IL 1α, eine Rolle spielt. Es kann zur IL-8-Bildung durch die Talgdrüsenzellen kommen und somit zu hemotaktischer Bewegung von Entzündungszellen zur Talgdrüse. Somit wurde im Rahmen der Erfindung festgestellt, dass Spezifische 5-Lixygenase-Hemmer bzw. -Inhibitoren als ausgezeichnete Wirkstoffe zur Behandlung der Akne, insbesondere der entzündlichen Akne, eingesetzt werden können.

Bisher sind Leukotriene, die eine Familie von Lipid-Mediatoren mit einer Vielzahl von pharmakologischen Wirkungen auf das respiratorische, das cardiovaskuläre, das gastrointestinale und das kutane System haben, als Ziel anderer pathophysiologischer Vorgänge in Tiermodellen und beim Menschen in Betracht gezogen worden, wobei Krankheitszustände wie Asthma, adulte RDS (Respiratory Distress Syndrom), chronische Bronchitis, septischer Schock, entzündliche Darmkrankheit, Krebs, Dermatitis, systemischer Lupus erythetodes und Psoriasis im Vordergrund standen (s. Lotti TM, Menchini G, Spallanzani A et al. Arachidonate transforming and immunomodulating agents: unapproved uses or indications. Clin Dermatol 2000; 18:118-123; Zhu YI, Stller MJ: Preview of potential therapeutic applications of leukotriene B4 inhibitors in Dermatology. Skin Pharmacol Appl Skin Physiol 2000; 13:235-295).

Erfindungsgemäß werden die in Anspruch 1 genannten 5-Lipoxygenase-Inhibitoren verwendet.

Gemäß der Erfindung einsetzbare, bekannte 5-Lipoxygenase-Inhibitoren schließen ein:

Masoprocol, Tenidap, (±)-1=(1-benzo[b]thien-2-ylethyl)-1-Hydroxyharnstoff (Zileuton, Abbott A-64077) Abbott A-76745, N'-[[5-(4-Fluorophenoxy)furan-2yl]-1-methyl-2-propynyl]-N'-Hydroxyharnstoff (Abbott A-78773), (R)(+)N'-[[5-(4-Fluorophenoxy)furan-2-yl]-1-methyl-2-propynyl]-N-hydroxyharnstoff (Abbott A-79175), Abbott ABT 761, Dainippon AL-3264, Bayer Bay-x-1005, Biofor BF-389, bunaprolast, Ciba-Geigy CGS-25997, Cytomed CMI-392, Cytomed CMI-568, Atlantic Pharmaceutical CT3, Takeda CV-6504, Efamol EF-40, Enazadrem-Phosphat, Leo Denmark ETH-615, Flezelastin-Hydrochlorid, Flobufen, Merck Frosst L 663536, Merckle ML3000, Linazolast, Lonapalen, Mercian MER W8020, N-Hydroxy-N[1-(2-phenyl-5-benzofuranyl)ethyl]harnstoff (R. W. Johnson Research Institute), Ontazolast, 3M Pharmaceuticals R-840, Rilopirox, Hoechst Marion Roussel RU54808, Schering Plough SCH 40120, Tepoxalin, Tanabe 757, Tanabe 799, Linetastine (Terumo, TMK-688), Glaxo Wellcome WILD20, Zeneca ZD-2138, Abbott A-121798, Abbott A 72694, Abbott A-80263, Biofor BF-397, Bristol-Myers Squibb BU-4601A, Carbazoycin C, Lagunamycin, Wellcome BW-70C, Ciba-Geigy CGS-26529, Warner-Lambert CI 1004, Warner-Lambert PD-136005, Warner-Lambert PD-145246, Eisai E 3040, Fujirebio F-1322, Fisons FPL-64170, Fujisawa FR 110302, Nippon Hypox HX 0386, Merck & Co L-699333, Merck Frosst L 739010, Lilly LY269415, Lilly LY 178002, Meiji Milk MM-7002, Hoechst Roussel P 8892, Hoechst Roussel P 8977, Hoechst Roussel HP977, SmithKline Beecham SB-202235, Green Cross SS-81-OH, Terumo Keio University TMK 685, American Home Products WAY-121520, American Home Products WAY-125007, Zeneca ZD 7717, Zeneca ZM 216800, Zeneca ZM 230487, 1,2-Dihydro-n-(2-thiazolyl)-1-oxopyrrolo(3,2,1-kl)pheno-thiazin-1-carboxamid, Abbott A-65260, Abbott A-69412, Abbott-63162, American Home Products AHR-5333, Bayer Bay-q-1531, Boehringer Ingelheim BI-L-357, Boehringer Ingelheim BI-L-93BS, Boehringer Ingelheim BI-L 226XX, Bristol-Myers Squibb BMY-30094, Carbazomycin B, Wellcome BW 4C, Wellcome BW-B218C, Wellcome BW-B70C, Chauvin CBS-1114, Ciba-Geigy CGS-21595, Ciba-Geigy CGS-22745, CibaGeigy CGS-23885, Ciba-Geigy CGS 24891, Ciba-Geigy CGS-8515, Chiesi CHF-1909, Warner-Lambert CI-986, Warner-Lambert CI 987, Cirsiliol, Docebenon, DuPont Merck DuP-654, Eisai E 5110, Eisai E-6080, Green Cross EN-105, Enofelast, Epocarbazolin-A, Eprovafen, Evandamin, Forsythiasid, Fisons FPL 62064, Glaxo GR-80907, Zeneca ICI-211965, Isoflavane, Kyowa Hakko KF-8940, Merck & Co L-651392, Merck & Co L651896, Merck & Co L-652343, Merck & Co L-656224, Merck & Co L-670630, Merck & Co L-674636, Merck & Co L-691816, Lilly LY233569, Lilly LY-280810, Merck & Co MK-591, Merck & Co MK886, Nitrosoxacin-A, Ono ONO-5349, Ono ONO-LP-219, Ono ONOLP-269, Warner-Lambert PD-127443, Purdue Frederick PF-5901, Sandoz QA-208-199, Johnson & Johnson R-68151, Johnson & Johnson R-85355, Rhone-Poulenc Rorer Rev-5367, Revlon 5901, Rhone-Poulenc Rorer RG-5901-A, Rhone-Poulenc Rorer RG-6866, Roussel-Uclaf RU-46057, Searle SC-41661A, Searle SC-45662, Sandoz SDZ-210610, SmithKline Beecham SK&F-104351, SmithKline Beecham SK&F-104493, SmithKline Beecham SK&F-105809, Synthelabo SL-810433, Teijin TEI-8005, Terumo TMK-777, Terumo TMK-781, Terumo TMK-789, Terumo TMK-919, Terumo TMK-992, Teikoku Hormone TZI-2721, Teikoku Hormone TZI-41127, American Home Products WAY-120739, American Home Products WY 47288, American Home Products WY-48252, American Home Products WY-50295, Yoshitomi Y-19432, Dihydroarachidonsäure, Merck MK571, Merck MK679, ICI207,968 und ICI204,219 (ICI), SC-41930, SC-51146, SC-37920, SC-53228, SC-50605 und SC-51146 (Searle), Wako AA-681, Wellcome BW755C, KC11404 [(4-Methyl-2-pyridinyl)-1-piperazinyl)ethyl)-4H-pyrrolo(3,2,1-ij)chinolin, 15-HETE, Leflunomide (HWA486), 4-Acylaminophenyl-Derivate, Chamazulen (Chamomilextrakt), polyungesättigte Fettsäuren, VLM295 oder LY293111 (Vanguard), 4,5-dihydro-1H-1,2,4-Triazolchinon-Addukte des Typs 1a, b, c (N-Addukte) bzw. des Typs 2 (C-Addukte) als Produkte der Reaktion von Chinonen with N-Alkyl- und N-Arylhydrazonen, und Terpene mit Leitstruktur Acetyl-11-keto-beta-Boswelliasäure (AKBA) (Nonredox-Inhibitoren).

Von diesen 5-Lipoxygenase-Inhibitoren sind im Hinblick auf eine bessere Wirksamkeit solche bevorzugt, die vom nichtsteroidalen Typ sind. Besonders bevorzugt ist der 5-Lipoxygenase-Inhibitor (±)-1-(1.benzo[b]thien-2-ylethyl)-1-Hydroxyharnstoff (Zileuton; Abbott A-64077; Zyflo^{R}), weil damit eine minimale Toxizität sowie eine gute Verträglichkeit verbunden ist.

Beispiele für 12-Lipoxygenase-Inhibitoren, die gleichzeitig auch 5- Lipoxygenase Inhibitorwirkung besitzen, sind Luteolin und Chrysoerol.

Die in Anspruch 1 genannten Lipoxygenase-Inhibitoren können allein oder in Kombination von zwei oder mehreren Inhibitoren gleichen oder verschiedenen Lipoxygenase-Typs eingesetzt werden. Günstig kann sich auch eine Kombination von mindestens einem 5-Lipoxygenase-Inhibitor von Anspruch 1 mit mindestens einem anderen, herkömmlichen Anti-Akne-Wirkstoff oder -Mittel eingesetzt werden, bevorzugt in Kombination mit gegen Akne wirksamen Antibiotika (lokal oder systemisch), Azelainsäure (lokal), Benzoylperoxid (lokal) und insbesondere in Kombination mit einer Retinoidverbindung (lokal oder systemisch).

Die Verabreichung des Wirkstoffs kann über übliche Applikationswege erfolgen, z.B. auf systemische Art und Weise, enteral, insbesondere oral oder rektal, transdermal, nasal mittels Inhalation sowie parenteral, insbesondere mittels Injektion (subkutan, intramuskulär oder intravenös), oder dergleichen. Behandelt werden können Menschen und Tiere wie beispielsweise Säugetiere und Nager. Damit die systemische Beeinträchtigung durch den Wirkstoffeinsatz möglichst gering gehalten bzw. ausgeschlossen wird, ist die lokale und insbesondere die topische Applikation bevorzugt. Hierfür können trägerfreie oder auf Trägern wie Pflaster, Verbandsmaterial etc. basierende System angewandt werden. Eine besonders bevorzugte Kombinationstherapie besteht in der Applikation von in Anspruch 1 genannten 5-Lipoxygenase-Inhibitoren mit Retinoiden, geeigneterweise jeweils gemeinsam lokal oder den 5-Lipoxygenase-Inhibitor systemisch und die Retinoidverbindung lokal. Ein Vorteil dieser Kombination besteht darin, dass Retinoide auf die Komedonen, die von den 5-Lipoxygenase-Inhibitoren nicht beeinflußt werden, wirken und damit durch die Kombination sowohl entzündliche als auch nicht entzündliche Läsionen gebessert werden können.

Die Lipoxygenase-Inhibitoren nach Anspruch 1 können in pharmazeutischen Zusammensetzungen zum Einsatz kommen, die eine gegen Akne wirksame Menge des Lipoxygenase-Inhibitors, ggf. wie beschrieben in Kombination mit anderen Anti-Akne-Mitteln, in Verbindung bzw. Beimengung mit für die jeweiligen oben bezeichneten Applikationsarten üblichen, pharmazeutisch akzeptablen Arzneimittelträgern umfassen. Geeignet sind beispielsweise Tabletten oder Kapseln mit dem bzw. den wirksamen Bestandteil(en) zusammen mit Streckmitteln wie z.B. Lactose, Dextrose, Sucrose, Manitol, Sorbitol, Cellulose und/oder Glycin, Gleitmitteln wie z.B. Siliciumdioxid, Talg, Stearinsäure sowie dessen Magnesium- oder Calciumsalze und/oder Polyethylenglycol, für Tabletten ferner Bindemittel wie z.B. Magnesiumaluminiumsilikat, Stärke, Gelatine, Tragakant, Methylcellulose, Natriumcarboxymethylcellulose und/oder Polyvinylpyrrolidon, sowie, falls gewünscht, Desintegriermittel wie z.B. Stärke bzw. modifizierte Stärke, Agar, Algeininsäure bzw. dessen Natriumsalz, oder Mischungen davon, und ggf. Absorbentien, Färbemittel, Geschmacksstoffe und Süßstoffe. Injektionsfähige Zusammensetzungen sind beispielsweise wässrige isotonische Lösungen oder Suspensionen, und Suppositorien werden vorzugsweise aus Fettemulsionen oder -Suspensionen hergestellt. Lokale bzw. topische pharmazeutische Zusammensetzungen sind beispielsweise Salben, Cremes, Gele, Öle, Emulsionen, Lotios, Pasten oder Lösungen und enthalten neben den Wirkstoffen die für die genannten Formulierungen entsprechenden Zusatzstoffe. Bei lokal topischen Applikationen können ferner Mittel zur Steigerung der perkutanen Resorption zugesetzt werden, beispielsweise Hyaluronidate, Dimethylsulfoxid (DMSO) und dergleichen. Die genannten pharmazeutischen Zusammensetzungen können sterilisiert sein und/oder weitere Hilfsstoffe enthalten, wie Konservierungsmittel, Stabilisatoren, Benetzungsmittel, Emulgatoren usw. Die Zusammensetzungen können mittels herkömmlicher Verfahren zum Mischen, Granulieren oder Beschichten hergestellt werden. Der bzw. die aktiven Wirkstoffe können in den Zusammensetzungen in einer Menge von 0,1 bis 50 Gew.-%, vorzugsweise 1 bis 40, weiter bevorzugt bis 20 Gew.-% und insbesondere bis 10 Gew.-%, bezogen auf das Gesamtgewicht der pharmazeutischen Zusammensetzungen, enthalten sein.

Die vorliegende Erfindung wird nachfolgend anhand eines Beispiels näher erläutert.

### Beispiel

Zur Behandlung der entzündlichen Akne wurden 10 Patienten mit Akne papulopustolosa (m:f 6:4, Alter 19 ± 5 Jahre) oral mit einem selektiven 5-Lipoxygenasehemmer, und zwar mit (±)-1-(1.benzo[b]thien-2-ylethyl)-1-Hydroxyharnstoff (Zileuton) 4x600 mg/d p.o. für 3 Monate behandelt. Die Läsionenzahl und der allgemeine Schweregradindex nach Allen und Schmidt (Allen BS, Smith JG: Various parameters for grading acne. Arch Dermatol. 1982; 118: 23-25) wurden klinisch beurteilt. Darüber hinaus wurden die Oberflächenlipide durch Sebumeter® und die Leberenzyme im Serum am Anfang der Studie, an der 2., 4., 8. und 12. Behandlungswoche und 2 Wochen nach Beendigung der Therapie bestimmt. Der LTB4-Nachweis im Blut durch Radioimmunassay und die Lipidfraktionen im Serum durch Chromatographie wurden am Anfang und in der 12. Behandlungswoche untersucht. Die Patienten wurden am Anfang und am Ende der Behandlung fotografiert. Der Akne-Schweregradindex nahm kontinuierlich und zeitabhängig ab (41 ± 28% des Initialwertes in der 12. Behandlungswoche; p<0.05). Dieser Effekt entstand aufgrund der Abnahme der Zahl der entzündlichen Läsionen bis zu 29 ± 24% (p<0.01), während Komedonen nicht ansprachen. Weder subjektive noch objektive Nebenwirkungen wurden registriert. Die Gesamtlipide im Sebum nahmen signifikant ab (35 ± 51%, p<0.05), auch nahmen die pro-inflammatorischen freien Fettsäuren (22 ± 18%) und die Lipoperoxide (26 ± 30%) ab. Der Grad der klinischen Besserung korrelierte stark mit der Reduktion der Gesamtlipide im Sebum (p = 0.0009, r²=0.81) und der freien Fettsäure (p=0,0003, r²=0,82). Im Gegensatz dazu waren LTB₄ im Blut und die Oberflächenlipide nicht beeinflusst. Alle untersuchten Parameter blieben praktisch unverändert während der zweiwöchigen Anschlussphase. Zusammenfassend konnten damit erste indirekte Beweise für eine genuine entzündliche Ätiologie der Akne erbracht werden. Darüber hinaus führte die systemische Hemmung des Arachidonsäure-Metabolismus zur Abnahme der Gesamtlipide und der pro-inflammatorischen Lipidfraktionen im Sebum, welche als verantwortlich für die Entwicklung der entzündlichen Akneläsionen betrachtet werden (Brom J, Konig W. Cytokine-induced (interleukins -3, -6 and -8 and tumor necrosis factor-beta) activation and deactivation of human neutrophils: Immunology. 1992; 75: 281-285 und Doran TI, Baff R, Jacobs P, Pacia E: Characterization of human sebaceous cells in vitro. J Invest Dermatol. 1991; 96: 341-348).

Folglich ist es gemäß der Erfindung durch Verwendung von den in Anspruch 1 genannten 5-Lipoxygenase-Inhibitoren möglich, eine signifikante Verbesserung der Akne-Symptome wirksam herbeizuführen, während nicht-inflammatorische Läsionen nicht beeinträchtigt werden. Gleichzeitig wird eine Verminderung Talgdrüsenlipide bei gleichzeitiger Inhibierung pro-entzündlicher Sebumlipide erreicht. Andererseits wirkt sich die fehlende Beeinflussung des LTB₄-Spiegels im Blut vorteilhaft aus, weil es auf fehlende systemische Effekte hinweist. Tatsächlich waren bei allen behandelten Patienten keine negativen Reaktionen beobachtet worden.

## Patentansprüche

1. Verwendung mindestens einer aus der Gruppe der 5-Lipoxygenase-Inhibitoren ausgewählten Substanz zur Herstellung einer pharmazeutischen Zusammensetzung zur therapeutisch wirksamen Behandlung der Akne, wobei als 5-Lipoxygenase-Inhibitor mindestens eine Substanz verwendet wird, die aus der folgenden Gruppe ausgewählt wird:
Masoprocol, Tenidap, (±)-1-(1-benzo[b]thien-2-ylethyl)-1-Hydroxyharnstoff (Zileuton, Abbott A-64077) Abbott A-76745, N'-[[5-(4-Fluorophenoxy)furan-2y1]-1-methyl-2-propynyl]-N'-Hydroxyharnstoff (Abbott A-78773),
(R)(+)N'-[[5-(4-Fluorophenoxy)furan-2-yl]-1-methyl-2-propynyl]-N-hydroxyharnstoff (Abbott A-79175), Abbott ABT 761, Dainippon AL-3264, Bayer Bay-x-1005, Biofor BF-389, bunaprolast, Ciba-Geigy CGS-25997, Cytomed CMI-392, Cytomed CMI-568, Atlantic Pharmaceutical CT3, Takeda CV-6504, Efamol EF-40, Enazadrem-Phosphat, Leo Denmark ETH-615, Flezelastin- Hydrochlorid, Flobufen, Merck Frosst L 663536, Merckle ML3000, Linazolast, Lonapalen, Mercian MER W8020, N-Hydroxy-N[1-(2-phenyl-5-benzofuranyl)ethyl]harnstoff (R. W. Johnson Research Institute), Ontazolast, 3M Pharmaceuticals R-840, Rilopirox, Hoechst Marion Roussel RU54808, Schering Plough SCH 40120, Tepoxalin, Tanabe 757, Tanabe 799, Linetastine (Terumo, TMK-688), Glaxo Wellcome WILD20, Zeneca ZD-2138, Abbott A-121798, Abbott A 72694, Abbott A-80263, Biofor BF-397, Bristol-Myers Squibb BU-4601A, Carbazoycin C, Lagunamycin, Wellcome BW-70C, Ciba-Geigy CGS-26529, Warner-Lambert CI 1004, Warner-Lambert PD-136005, Warner-Lambert PD-145246, Eisai E 3040, Fujirebio F-1322, Fisons FPL-64170, Fujisawa FR 110302, Nippon Hypox HX 0386, Merck & Co L-699333, Merck Frosst L 739010, Lilly LY269415, Lilly LY 178002, Meiji Milk MM-7002, Hoechst Roussel P 8892, Hoechst Roussel P 8977, Hoechst Roussel HP977, SmithKline Beecham SB-202235, Green Cross SS-81-OH, Terumo Keio University TMK 685, American Home Products WAY-121520, American Home Products WAY-125007, Zeneca ZD 7717, Zeneca ZM 216800, Zeneca ZM 230487, 1,2-Dihydro-n-(2-thiazolyl)-1-oxopyrrolo (3,2,1-kl)pheno-thiazin-1-carboxamid, Abbott A-65260, Abbott A-69412, Abbott-63162, American Home Products AHR-5333, Bayer Bay-q-1531, Boehringer Ingelheim BI-L-357, Boehringer Ingelheim BI-L-93BS, Boehringer Ingelheim BI-L 226XX, Bristol-Myers Squibb BMY-30094, Carbazomycin B, Wellcome BW 4C, Wellcome BW-B218C, Wellcome BW-B70C, Chauvin CBS-1114, Ciba-Geigy CGS-21595, Ciba-Geigy CGS-22745, CibaGeigy CGS-23885, Ciba-Geigy CGS 24891, Ciba-Geigy CGS-8515, Chiesi CHF-1909, Warner-Lambert CI-986, Warner-Lambert CI 987, Cirsiliol, Docebenon, DuPont Merck DuP-654, Eisai E 5110, Eisai E-6080, Green Cross EN-105, Enofelast, Epocarbazolin-A, Eprovafen, Evandamin, Forsythiasid, Fisons FPL 62064, Glaxo GR-80907, Zeneca ICI-211965, Isoflavane, Kyowa Hakko KF-8940, Merck & Co L-651392, Merck & Co L651896, Merck & Co L-652343, Merck & Co L-656224, Merck & Co L-670630, Merck & Co L-674636, Merck & Co L-691816, Lilly LY233569, Lilly LY-280810, Merck & Co MK-591, Merck & Co MK886, Nitrosoxacin-A, Ono ONO-5349, Ono ONO-LP-219, Ono ONOLP-269, Warner-Lambert PD-127443, Purdue Frederick PF-5901, Sandoz QA-208-199, Johnson & Johnson R-68151, Johnson & Johnson R-85355, Rhone-Poulenc Rorer Rev-5367, Revlon 5901, Rhone-Poulenc Rorer RG-5901-A, Rhone-Poulenc Rorer RG-6866, Roussel-Uclaf RU-46057, Searle SC-41661A, Searle SC-45662, Sandoz SDZ-210610, SmithKline Beecham SK&F-104351, SmithKline Beecham SK&F-104493, SmithKline Beecham SK&F-105809, Synthelabo SL-810433, Teijin TEI-8005, Terumo TMK-777, Terumo TMK-781, Terumo TMK-789, Terumo TMK-919, Terumo TMK-992, Teikoku Hormone TZI-2721, Teikoku Hormone TZI-41127, American Home Products WAY-120739, American Home Products WY 47288, American Home Products WY-48252, American Home Products WY-50295, Yoshitomi Y-19432, Dihydroärachidonsäure, Merck MK571, Merck MK679, ICI207,968 und ICI204,219 (ICI), SC-41930, SC-51146, SC-37920, SC-53228, SC-50605 und SC-51146 (Searle), Wako AA-681, Wellcome BW755C, KC11404 [(4-Methyl-2-pyridinyl)-1-piperazinyl)ethyl)-4H-pyrrolo(3,2,1-ij)chinolin, 15-HETE, Leflunomide (HWA486), 4-Acylaminophenyl-Derivate, Chamazulen (Chamomilextrakt), polyungesättigte Fettsäuren, VLM295 oder LY293111 (Vanguard), 4,5-dihydro-1H-1,2,4-Triazolchinon-Addukte des Typs 1a, b, c (N-Addukte) bzw. des Typs 2 (C-Addukte) als Produkte der Reaktion von Chinonen with N-Alkyl- und N-Arylhydrazonen, und
Terpene mit Leitstruktur Acetyl-11-keto-beta-Boswelliasäure (AKBA).

2. Verwendung gemäß Anspruch 1, wobei ein nichtsteroidaler Inhibitor verwendet wird.

3. Verwendung gemäß Anspruch 1, wobei der 5-Lipoxygenase-Inhibitor aus der Gruppe bestehend aus Zileuton ausgewählt wird.

4. Verwendung gemäß einem der vorangehenden Ansprüche, wobei der Lipoxygenase-Inhibitor mit mindestens einem weiteren, herkömmlichen Anti-Akne-Wirkstoff kombiniert wird.

5. Verwendung gemäß Anspruch 4, wobei ein 5-Lipoxygenase-Inhibitor und ein Retinoid kombiniert werden.

6. Verwendung gemäß einem der vorangehenden Ansprüche, wobei eine orale und/oder topische Applikation erfolgt.

7. Verwendung gemäß einem der vorangehenden Ansprüche, wobei die entzündliche Akne behandelt wird.

8. Verwendung gemäß einem der vorangehenden Ansprüche, wobei die pharmazeutische Zusammensetzung mindestens einen 5-Lipoxygenase-Inhibitor und einen pharmazeutisch akzeptablen Träger umfasst.

## Claims

1. Use of at least one substance selected from the group comprising the 5-lipoxygenase inhibitors for the preparation of a pharmaceutical composition for therapeutically effective treatment of acne, wherein at least one substance selected from the following group is used as 5-lipoxygenase inhibitor:
Masoprocol, Tenidap, (±)-1-(1-benzo[b]thiene-2-ylethyl)-1-hydroxyurea (Zileuton, Abbott A-64077) Abbott A-76745,
N'-[[5-(4-fluorophenoxy)furane-2yl]-1-methyl-2-propynyl]-N'-hydroxyurea (Abbott A-78773), (R)(+)N'-[[5-(4-fluorophenoxy)furane-2yl]-1-methyl-2-propynyl]-N-hydroxyurea (Abbott A-79175), Abbott ABT 761, Dainippon AL-3264, Bayer Bay-x-1005, Biofor BF-389, bunaprolast, Ciba-Geigy CGS-25997, Cytomed CMI-392, Cytomed CMI-568, Atlantic Pharmaceutical CT3, Takeda CV-6504, Efamol EF-40, Enazadrem-phosphate, Leo Denmark ETH-615, Flezelastin-hydrochloride, Flobufen, Merck Frosst L 663536, Merckle ML3000, Linazolast, Lonapalen, Mercian MER W8020,
N-hydroxy-N[1-(2-phenyl-5-benzofuranyl)ethyl]urea (R. W. Johnson Research Institute), Ontazolast, 3M Pharmaceuticals R-840, Rilopirox, Hoechst Marion Roussel RU54808, Schering Plough SCH 40120, Tepoxalin, Tanabe 757, Tanabe 799, Linetastine (Terumo, TMK-688), Glaxo Wellcome WILD20, Zeneca ZD-2138, Abbott A-121798, Abbott A 72694, Abbott A-80263, Biofor BF-397, Bristol-Myers Squibb BU-4601A, Carbazomycin C, Lagunamycin, Wellcome BW-70C, Ciba-Geigy CGS-26529, Warner-Lambert Cl 1004, Wamer-Lambert PD-136005, Wamer-Lambert PD-145246, Eisai E 3040, Fujirebio F-1322, Fisons FPL-64170, Fujisawa FR 110302, Nippon Hypox HX 0386, Merck & Co L-699333, Merck Frosst L 739010, Lilly LY269415, Lilly LY 178002, Meiji Milk MM-7002, Hoechst Roussel P 8892, Hoechst Roussel P 8977, Hoechst Roussel HP977, SmithKline Beecham SB-202235, Green Cross SS-81-OH, Terumo Keio University TMK 685, American Home Products WAY-121520, American Home Products WAY-125007, Zeneca ZD 7717, Zeneca ZM 216800, Zeneca ZM 230487, 1,2-dihydro-n-(2-thiazolyl)-1-oxypyrrolo(3,2,1-kl)pheno-thiazine-1-carboxamide, Abbott A-65260, Abbott A-69412, Abbott-63162, American Home Products AHR-5333, Bayer Bay-q-1531, Boehringer Ingelheim BI-L-357, Boehringer Ingelheim BI-L-93BS, Boehringer Ingelheim BI-L 226XX, Bristol-Myers Squibb BMY-30094, Carbazomycin B, Wellcome BW 4C, Wellcome BW-B218C, Wellcome BW-B70C, Chauvin CBS-1114, Ciba-Geigy CGS-21595, Ciba-Geigy CGS-22745, Ciba-Geigy CGS-23885, Ciba-Geigy CGS 24891, Ciba-Geigy CGS-8515, Chiesi CHF-1909, Wamer-Lambert CI-986, Warner-Lambert CI 987, Cirsiliol, Docebenon, DuPont Merck DuP-654, Eisai E 5110, Eisai E-6080, Green Cross EN-105, Enofelast, Epocarbazolin-A, Eprovafen, Evandamin, Forsythiasid, Fisons FPL 62064, Glaxo GR-80907, Zeneca ICI-211965, lsoflavane, Kyowa Hakko KF-8940, Merck & Co L-651392, Merck & Co L651896, Merck & Co L-652343, Merck & Co L-656224, Merck & Co L-670630, Merck & Co L-674636, Merck & Co L-691816, Lilly LY233569, Lilly LY-280810, Merck & Co MK-591, Merck & Co MK886, Nitrosoxacin-A, Ono ONO-5349, Ono ONO-LP-219, Ono ONOLP-269, Warner-Lambert PD-127443, Purdue Frederick PF-5901, Sandoz QA-208-199, Johnson & Johnson R-68151, Johnson & Johnson R-85355, Rhone-Poulenc Rorer Rev-5367, Revlon 5901, Rhone-Poulenc Rorer RG-5901-A, Rhone-Poulenc Rorer RG-6866, Roussel-Uclaf RU-46057, Searle SC-41661A, Searle SC-45662, Sandoz SDZ-210610, SmithKline Beecham SK&F-104351, SmithKline Beecham SK&F-104493, SmithKline Beecham SKBF-105809, Synthelabo SL-810433, Teijin TEI-8005, Terumo TMK-777, Terumo TMK-781, Terumo TMK-789, Terumo TMK-919, Terumo TMK-992, Teikoku Hormone TZI-2721, Teikoku Hormone TZI-41127, American Home Products WAY-120739, American Home Products WY 47288, American Home Products WY-48252, American Home Products WY-50295, Yoshitomi Y-19432, dihydroarachidonic acid, Merck MK571, Merck MK679, ICI207,968 and IC1204,219 (ICI), SC-41930, SC-51146, SC-37920, SC-53228, SC-50605 and SC-51146 (Searle), Wako AA-681, Wellcome BW755C, KC11404 [(4-methyl-2-pyridinyl)-1-piperazinyl)ethyl)-4H-pyrrolo(3,2,1-ij)quinoline, 15-HETE, Leflunomide (HWA486), 4-acylaminophenyl derivatives, chamazulene (camomile extract), polyunsaturated fatty acids, VLM295 or LY293111 (Vanguard), 4,5-dihydro-1H-1,2,4-triazolquinone adducts of Type 1a, b, c (N-adducts) or of Type 2 (C-adducts) as products of the reaction of quinones with
N-alkyl and N-aryl hydrazones, and terpenes with the lead compound acetyl-11-keto-beta-boswellic acid (AKBA).

2. Use according to Claim 1, wherein a non-steroidal inhibitor is used.

3. Use according to Claim 1, wherein the 5-lipoxygenase inhibitor is selected from the group consisting of Zileuton.

4. Use according to any one of the preceding claims, wherein the lipoxygenase inhibitor is combined with at least one other conventional anti-acne active substance.

5. Use according to Claim 4, wherein a 5-lipoxygenase inhibitor and a retinoid are combined.

6. Use according to any one of the preceding claims, wherein oral and/or topical application is performed.

7. Use according to any one of the preceding claims, wherein inflammatory acne is treated.

8. Use according to any one of the preceding claims, wherein the pharmaceutical composition contains at least a 5-lipoxygenase inhibitor and a pharmaceutically acceptable carrier.

## Revendications

1. Utilisation d'au moins une substance choisie dans le groupe des inhibiteurs de 5-lipoxygénase pour la préparation d'une composition pharmaceutique pour le traitement thérapeutiquement efficace de l'acné, dans laquelle au moins une substance choisie dans le groupe ci-après est utilisée comme inhibiteur de 5-lipoxygénase :
Masoprocol, Tenidap, (±)-1-(1-benzo[b]thièn-2-yléthyl)-1-hydroxyurée (Zileuton, Abbott A-64077) Abbott A-76745, N'-[[5-(4-fluorophénoxy)furan-2yl]-1-méthyl-2-propynyl]-N'-hydroxyurée (Abbott A-78773), (R)(+)N'-[[5-(4-fluorophénoxy)furan-2-yl]-1-méthyl-2-propynyl]N- hydroxyurée (Abbott A-79175), Abbott ABT-761, Dainippon AL-3264, Bayer Bay-x-1005, Biofor BF-389, bunaprolast, Ciba-Geigy CGS-25997, Cytomed CMI-392, Cytomed CMI-568, Atlantic Parmaceutical CT3, Takeda CV-6504, Efamol EF-40, Enazadrem phosphate, Leo Denmark ETH-615, Flezelastin chlorhydrate, Flobufen, Merck Frosst L 663536, Merckle ML3000, Linzolast, Lonapalen, Mercian MER W8020, N-hydroxy-N[1-(2-phényl-5-benzofuranyl)éthyl]urée, (R. W. Johnson Research Institute), Ontazolast, 3M Parmaceuticals R-840, Rilopirox, Hoechst Marion Roussel RU54808, Schering Plough SCH 40120, Tepoxalin, Tanabe 757, Tanabe 799, Linetastine (Terumo, TMK-688), Glaxo Wellcome WILD20, Zeneca ZD-2138, Abbott A-121798, Abbott A 72694, Abbott A-80263, Biofor BF-397, Bristol-Myers Squibb BU-4601A, Carbazoycine C, Lagunamycine, Wellcome BW-70C, Ciba-Geigy CGS-26529, Warner-Lambert CI 1004, Warner-Lambert PD-136005, Warner-Lambert PD-145246, Eisai E 3040, Fujirebio F-1322, Fisons FPL-64170, Fujisawa FR 110302, Nippon Hypox HX 0386, Merck & Co L-699333, Merck Frosst L 739010, Lilly LY269415. Lilly LY 178002, Meiji Milk MM-7002, Hoechst Roussel P 8892, Hoechst Roussel P 8977, Hoechst Roussel HP977, SmithKline Beecham SB-202235, Green Cross SS-81-OH, Terumo Keio University TMK 685, American Home Products WAY-121520, American Home Products WAY-125007, Zeneca Zd 7717, Zeneca ZM 216800, Zeneca ZM 230487, 1,2-dihydro-n-(2-thiazolyl)-1-oxopyrrolo(3,2,1-kl)phénothiazine-1-carboxamide, Abbott A-65260, Abbott A-69412, Abbott 63162, American Home Products AHR-5333, Bayer Bay-q-1531, Boehringer Ingelheim BI-L-357, Boehringer Ingelheim BI-L-93BS, Boehringer Ingelheim BI-L 226XX, Bristol-Myers Squibb BMY-30094, Carbazomycin B, Wellcome BW 4C, Wellcome BW-B218C, Wellcome BW-B70C, Chauvin CBS-1114, Ciba-Geigy CGS-21595, Ciba-Geigy CGS-22745, Ciba-Geigy CGS-23885, Ciba-Geigy CGS-24891, Ciba-Geigy CGS-8515, Chiesi CHF-1909,Warner-Lambert CI-986,Warner-Lambert CI-987, Circiliol, Docebenon, DuPont Merck DuP-654, Eisai E 5110, Eisai E 6080, Green Cross EN-105, Enofelast, Epocarbazoline-A, Eprovafen, Evandamine, Forsythiaside, Fisons FPL 62064, Glaxo GR-80907, Zeneca ICI-211965, Isoflavane, Kyowa Hakko KF-8940, Merk & Co L-651392, Merk & Co L-651896, Merk & Co L-652343, Merk & Co L-656224, Merk & Co L-670630, Merk & Co L-674636, Merk & Co L-691816, Lilly LY233569, Lilly LY-280810, Merk & Co MK-591, Merk & Co MK886, Nitrosoxycine-A, Ono ONO-5349, Ono ONO-LP-219, Ono ONO-LP-269, Warner-Lambert PD-127443, Purdue Frederick PF-5901, Sandoz QA-208-199, Johnson & Johnson R-68151, Johnson & Johnson R-85355, Rhône-Poulenc Rorer Rev-5367, Revlon 5901, Rhône-Poulenc Rorer RG-5901-A, Rhône-Poulenc Rorer RG-6866, Roussel-Uclaf RU-46057, Searle SC-41661A, Searle SC-45662,Sandoz SDZ-210610, SmithKline Beecham SK&F-104351, SmithKline Beecham SK&F-104493, SmithKline Beecham SK&F-105809, Synthelabo SL-810433, Teijin TEI-8005, Terumo TMK-777, Terumo TMK-781, Terumo TMK-789, Terumo TMK-919, Terumo TMK-992, Teikoku Hormone TZI-2721, Teikoku Hormone TZI-41127, American Home Products WAY-120739, American Home Products WY 47288, American Home Products WY-48252, American Home Products WY-50295, Yoshitomi Y-19432, acide dihydroarachidonique, Merck MK571, Merck MK679, ICI207,968 et ICI204,219 (ICI), SC-41930, SC-51146, SC-37920, SC-53228, SC-50605 et SC-51146 (searle), Wako AA-681, Wellcome BW755C, KC11404 [(4-méthyl-2-pyridinyl)-1-pipérazinyl)éthyl)-4H-pyrrolo(3,2,1-ij)quinoléine, 15-HETE, Leflunomide 8HWA486), dérivés 4-acylaminophénylés, chamazulène (extrait de camomille), acides gras poly-insaturés, VLM295 ou LY293111 (Vanguard), composés d'addition de la 4,5-dihydro-1H-1,2,4-triazolequinone des types 1a, b, c (N-addition), ou du type 2 (C-addition) comme produits de la réaction des quinones avec les N-alkyl- et N-arylhydrazones, et
terpènes de la structure générale de l'acide acétyl-11-céto-bêta-boswellique (AKBA).

2. Utilisation selon la revendication 1, dans laquelle un inhibiteur non stéroïdien est utilisé.

3. Utilisation selon la revendication 1, dans laquelle l'inhibiteur de 5-lipoxygénase est choisi dans le groupe consistant en le « Zileuton ».

4. Utilisation selon l'une des revendications précédentes, dans laquelle l'inhibiteur de lipoxygénase est combiné à au moins un autre principe actif anti-acné conventionnel.

5. Utilisation selon la revendication 4, dans laquelle un inhibiteur de 5-lipoxygénase et un rétinoide sont combinés.

6. Utilisation selon l'une des revendications précédentes, dans laquelle s'effectuent une application orale et/ou une application topique.

7. Utilisation selon l'une des revendications précédentes, dans laquelle on traite l'acné inflammatoire.

8. Utilisation selon l'une des revendications précédentes, dans laquelle la composition pharmaceutique comporte au moins un inhibiteur de 5-lipoxygénase et un support pharmaceutiquement acceptable.
